# EUROPEAN PATENT APPLICATION

(11) **EP 4 295 875 A1**
(43) Date of publication of application: **27.12.2023**
(21) Application number: 22179971.1
(22) Date of filing: 20.06.2022
(51) Int. Cl.: A61M 5/24

(54) **SIMPLIFIED COMMUNICATION INTERFACE FOR DRUG DELIVERY DEVICES**

(71) Applicant: Ypsomed AG, 3401 Burgdorf (CH)
(72) Inventor: Schüpbach, Simon, 3400 Burgdorf (CH)
(74) Representative: Meier Obertüfer, Jürg

(57) **Abstract**

The present invention is concerned with an injection device comprising electronics. The electronics comprise a nearfield communication unit which may be used on one side to read information from near field communication readable item related to a drug cartridge and on the other side to provide information the a further device.

## Description

The present invention relates to injection devices used for the subcutaneous application of fluid drugs and drug compositions.

### BACKGROUND OF THE INVENTION

Many complex drugs, like hormones and antibodies may not be administered orally but have to be applied by injection. The use of injection devices like insulin pens has been established decades ago. In recent years two influencing aspects have become more prominent: sustainability and connectivity. Waste and resource usage should be reduced to a minimum. On the other side, modern injection devices often comprise electronics and wireless connectivity using Bluetooth, Wireless LAN or near field communication. Electronics usually comprise valuable materials as well as potentially poisonous substances such that correct disposal is critical. One possibility to bring the somewhat contradictory aspects under one umbrella is to increase the lifespan of an injection device by making it at least partly reusable. Semi-disposable injection devices have been known for many years. One example is the OptiClik semi-disposable insulin pen developed by Ypsomed. However, most commercially available autoinjectors today are disposable injection devices.

In comparison with classical injection pens autoinjectors are more demanding to design, because of the driving setup. For many autoinjectors strong springs are used as driving means which provides the driving force to eject the content of a drug cartridge or syringe. In disposable autoinjectors the springs are typically pre-tensioned such that a patient using the autoinjector can easily operate the autoinjector. A modification of such a disposable device (made to be used once) to become reusable is challenging from a technical point of view as well as from the point of view of usability for patients. Re-tensioning a spring is burdensome for most patients. To keep a re-tensioned spring securely in the tensioned state repeatedly for many times requires a very stable construction of the autoinjector. As alternative, a motor may be used as a driving means, which makes sense for a device which is reusable.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system", "injector", or "autoinjector" refers to a device that is removed from the injection site after each medication event or drug delivery process, whereas the term "infusion system" refers to a device with a cannula or needle that remains in the skin of the patient for a prolonged period of time, for example, several hours.

### DESCRIPTION OF THE INVENTION

It is an objective of the invention to provide sustainable but modern injection devices and systems.

This objective is achieved by the injection device and the corresponding system according to the independent claims. Further beneficial embodiments and forms of the invention may be found in the dependent claims, the description and the figures.

One aspect of the invention relates to an injection device for injection of fluid medicaments into a patient's tissue. Examples of possible injection devices are injection pens, autoinjectors, patch injection devices or the like. The injection device according to the invention comprises electronics, with at least an electronic controller and a near field communication unit in functional connection to the controller. The injection device according to the invention further comprises a cartridge assembly. The cartridge assembly has to be construed broadly. In one possible arrangement the cartridge assembly is mainly constituted by the cartridge as such, where the cartridge may be formed by a prefilled syringe, ampule or the commonly used carpule. In an another arrangement the cartridge assembly may be constituted by a cartridge unit, e. g. comprising a housing, the cartridge as such, a needle protection sleeve, a needle assembly, and/or further constituents. It has to be understood that the cartridge assembly shall not be construed as a fully functional injection device of its own. So consequently, a driving arrangement shall not be part of the cartridge assembly even though particular elements of a driving arrangement may be located in the drive arrangement. The cartridge assembly comprises in any case a near field communication readable item fixedly attached to the cartridge assembly. The near field communication readable item, in a simple design according to the invention, is a RFID or NFC (smart) tag (according ISO/IEC 14443A/B, ISO/IEC15693, ISO/IEC18000-3, and/or according to any other suitable standard). Such tags may store information which may be read by a reading device. For example a tag according to the invention may store information regarding the medicament contained in the cartridge, the manufacturing date of the cartridge assembly and/or the medicament as well as any other information considered useful in the context of a therapy or in relation to the therapy. However, such tags may store a limited amount of data only, typically ranging from 48 Bytes to 1 Mbyte. E. g. the NTAG213 by NXP (www.nxp.com) comprises a user storage of 144 Bytes. According to the invention the near field communication unit of the injection device is capable of acquiring the data stored on the near field communication readable item. The cartridge assembly may be fixedly attached to the injection device. For example, the actual injection device may be manufactured at a first site, a prefilled syringe comprising the near field communication readable item (tag) as the cartridge assembly may be manufactured at a second site, and the end assembly, i. e. the mounting of the prefilled syringe, may take place at a third site. With the tag associated with the prefilled syringe it is not necessary to activate the injection device electronics during assembly, as all the necessary information may be obtained by the injection device at any point in time particularly also after assembly which simplifies the end assembly process significantly. Alternatively, the cartridge assembly may be releasably attachable to the injection device. In any case, if the cartridge assembly is attached to the injection device, the near field communication readable item and the near field communication unit are geometrically arranged at a distance to each other which allows for the stored information to be transferred from the item to the unit. The person skilled in the art is aware of appropriate geometrical arrangements for near field communication arrangements. The near field communication unit according the invention can be used in two modes. In a first mode, the near field communication unit is used to read information from the near field communication readable item. In the second mode, the near field communication unit is used to provide information to another device. The other device is a separate device, which comprises a near field communication reading device. For example, the near field communication unit may read information from the near field communication readable item, provide the read information to the controller and subsequently change mode to provide at least a fraction of the read information and/or further data to the separate device. The information provided may be based on the read information or on information created by the controller.

In advantageous embodiments the near field communication device may use one single antenna for sending and receiving data.

In advantageous embodiments the injection unit comprises only one wireless communication means, namely the near field communication unit, as described above.

In advantageous embodiments the other device as described above may act as a display device for the injection device, rendering moot the need for an alphanumeric display, like a LCD or an OLED display, arranged on the injection device.

In a further developed example, the near field communication device of the injection device may be used not only to read information from the cartridge but also from the other device, which is e. g. a smart phone. For example the smart phone may receive recall information regarding the medicament used in the injection device and transfer information via near field communication containing e. g. a list of recalled lot number to the injection device. In case the lot number of medicament currently used in the injection device matches an entry of the list, the injection device may alert the user and/or block the injection device. The alert may be first transferred to the smart phone and be displayed on the display of the smart phone. Another possibility of a data transfer from the other device (smart phone) to the injection device might relate to the transfer of a kind of therapy plan from smart phone to injection device.

The controller may monitor operation of the injection device according to the invention or it may control at least part of the operation of the injection device according to the invention.

One aspect of the invention relates to an injection device as described where near field communication means near field communication (NFC) according to the standard ISO/IEC 18092 and its normative references or any of related standards by the IEEE organization or related.

One aspect of the invention relates to a system combining the injection device according the invention and the separate device. The separate device may be a smart phone, a cellular phone, a personal digital assistant (PDA) like a palm pilot, a tablet computing device or any computing device with a near field communication reading device, like an NFC or a RFID reading device. Preferably, the separate device runs AndroidOS, iOS by Apple, Windows operating system, Linux operating system, MacOS operating system, or any related operating system capable of operating the near field communication reading device.

In one aspect of the invention the information provided by the near field communication unit of the injection device to the separate device is data relating to state information of the injection device or error information, both gathered by the controller. The error information may comprise error messages from the injection device as such, like operation failure, battery low state or the like. The error information may further or alternatively contain error information related to information acquired from the near field communication readable item, e. g. reached expiration date of the medicament, wrong medicament identification, or the like.

In one aspect of the invention the injection device according to the invention is a modular injection device. The modular injection device comprises at least a reusable module and a disposable module which may be releasably attached to the reusable module. The reusable module contains at least the controller and the near field communication unit. Disposable and reusable modules are attached to each other by snap, bayonet, threaded connections or the like. The disposable module comprises at least the cartridge assembly. After use or depletion of the cartridge, the disposable module is removed from the reusable module and disposed. A new disposable module may then be attached to the reusable module for further use.

In a more detailed example of the modular injection device according to the invention the injection device is preferably an autoinjector. The disposable unit is a cartridge unit. The reusable unit has a housing which houses at least a driving arrangement configured to force medicament out of a cartridge, electronics with a controller, and a near field communication unit. In, at, or on the housing a user interface is arranged preferably which is in functional communication with the controller. The cartridge unit comprises a housing in which the cartridge with the fluid medicament is arranged. At a surface of the housing of the cartridge unit a near field communication readable item, preferably a NFC tag, is arranged. The near field communication readable item stores information regarding the medicament, such as the type of medicament, the manufacturing date, information regarding shelf life and the like. Optionally, the tag may further comprise a temperature sensing element for logging of at least a maximum storage temperature or a humidity sensor.

The reusable unit's housing is configured to accommodate the cartridge unit at least partly within the housing with typically having the delivery end of the cartridge reaching out of a distal end of the reusable unit. The cartridge unit may releasably attached within the housing of the reusable unit for use. As the cartridge unit is accommodated in the reusable unit, the near field communication readable item of the cartridge unit gets located in the very proximity of the near field communication unit of the reusable unit. Driven by the controller, the near field communication may be used to read the information stored in the near field communication readable item. The read information may be used by the controller to influence operation of the injection device. Moreover, the information may be stored on the controller or a storage unit attached to the controller.

According to the invention the near field communication unit of the reusable unit of the injection device is not only configured to receive information but also to transmit information to other devices. In order to enable data transmission the controller is configured to set the near field communication unit into the so-called card emulation mode. Once in this mode the near field communication unit simulates a nearfield communication device from which information may be read, similar to the near field communication readable item as described above. One possibility for another device which reads or receives information from the injection device may a smart phone. The smart phone may be used to monitor a therapy and/or to give guidance to a person using the injection device.

In a further aspect the driving arrangement of the injection device comprises an electric or electronic drive train comprising an element movable along an axis of the injection device which is preferably an autoinjector to force medicament out of the cartridge. E. g. the movable element may be a piston rod applying force on a moveable piston in the cartridge, which in a preferred example may be a prefilled syringe. The movable element may by driven by an electric motor, like a stepper motor, a DC motor, or piezoelectric motor. A gearing setup may interconnect the motor and the movable element.

In a further aspect, electrical and/or electronic components of an injection device according to the invention are energized through an energizing element like a battery, an accumulator or a super cap, wherein the energizing element of the injection device is disposed within a housing or a housing part of the injection device. The energizing element may fixedly and unremovably disposed in the injection device. In an alternative the energizing element may removably disposed in the injection device, either for recycling purposes or for exchange during use of the injection device.

In a further aspect, an injection device according to the invention comprises a user interface. The user interface is operationally connected to the controller of the injection device. In a simple example the user interface consists of one or more LEDs (light emitting diodes), which may emit signals provided by the controller and meaningful to a person using the injection device. The signals may relate to a state of the injection device and/or to information received through near field communication from the cartridge or the cartridge unit.

In a more sophisticated example the user interface comprises a LCD or OLED Display capable displaying more comprehensive information regarding a device state and/or information regarding the cartridge.

Various described aspects in this disclosure relate to near field communication. Near field communication with regards to the present invention shall be construed in its broadest possible sense as a wireless communication method using radio frequency electro-magnetic radiation to transmit information over a distance of less than one centimeter to several centimeters.

### BRIEF DESCRIPTION OF THE DRAWINGS

The subject matter of the invention will be explained in more detail in the following text with reference to preferred exemplary embodiments which are illustrated in the attached drawings, in which:
- Fig.1: depicts a cartridge unit 10 configured to be inserted into a reusable unit of an injection device (autoinjector) 1 which may communicate with a smart phone 20;
- Fig.2: depicts the injection device 1 with it outer distal housing 1b removed revealing the near field communication unit 2; and
- Fig.3: depicts a longitudinal cross section of the injection device (autoinjector) 1 showing the cartridge unit 10 inserted in the injection device with the prefilled syringe 12. The further elements present in the injection device except the housing element are omitted in figure 3.

The reference symbols used in the drawings, and their primary meanings, are listed in summary form in the list of designations.

### DETAILED DESCRIPTION OF PREFERRED EMBODIMENTS

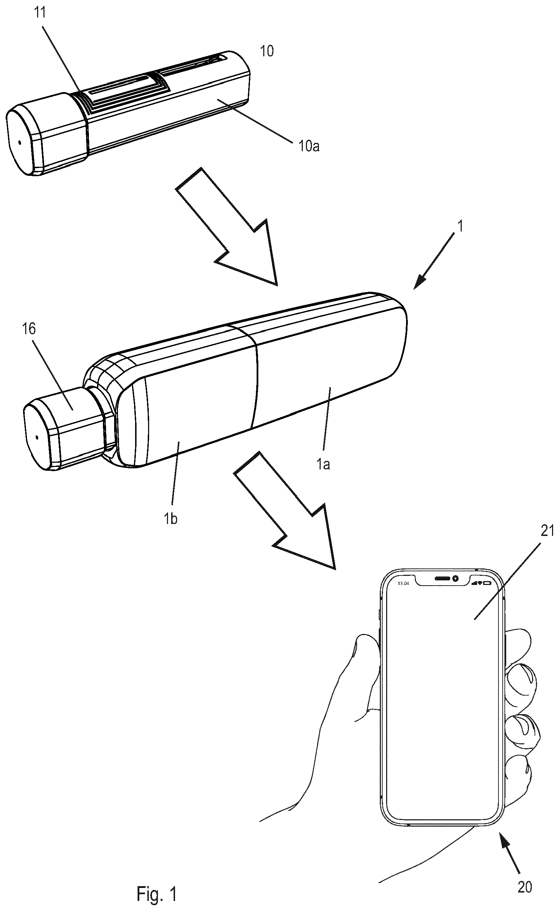

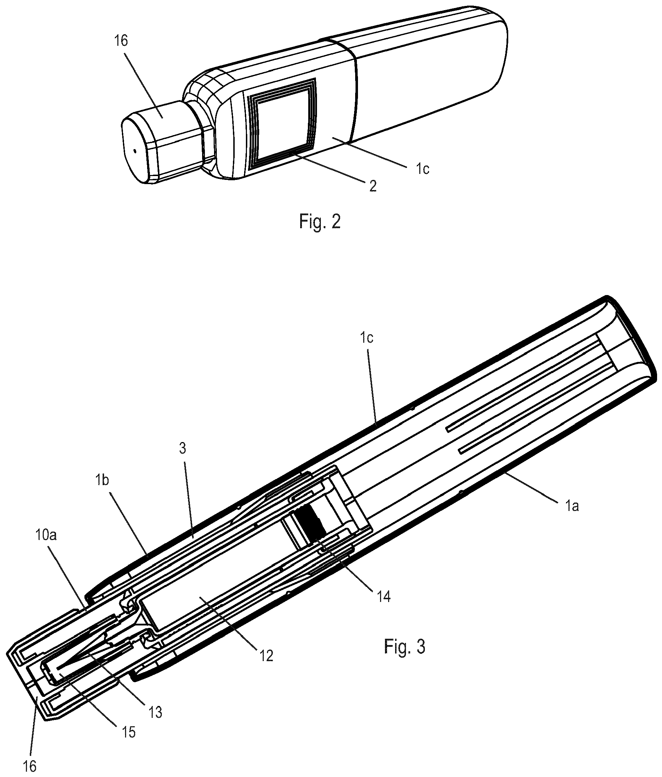

Figures 1 to 3 show an embodiment of the invention. Figure 1 shows a system according to the invention showing the injection device 1, an exchangeable cartridge unit 10 which may be inserted into the injection device 1, and smart phone 20. The NFC tag 11 on the cartridge unit housing 10a is depicted as a symbolic antenna. Figure 2 shows the injection device 1 with the outer distal housing 1b removed, such that the near field communication module, i. e. the NFC receiver and transmitter 2 becomes visible - also depicted as symbolic antenna. The cartridge module 10 comprises a prefilled syringe 12 (ref. figure 3). Within the syringe a piston 14 is disposed, which is movable between proximal position (as in figure 3) and a distal position where piston reaches the shoulder of the syringe body (not shown). As the piston is moved into distal direction, fluid medicament is ejected through the syringe cannula 13. Before use, the cannula is protected by a needle shield 15 which is removed by the needle shield removing unit 16 when the injection device 1 is to be used to deliver medicament.

Injection device 1 is a motor driven autoinjector. It comprises an electronic controller (not shown), a drive assembly comprising the motor (not shown) and the NFC receiver and transmitter. A person skilled in the art is aware of motor driven autoinjectors, e.g. US20200164145 A1 is a known example. The injection device 1 comprises an inner housing 1c and outer housings, namely the proximal outer housing 1a and distal outer housing. After insertion of the cartridge unit 10 is detected by the injection device, e. g. by sensors (not shown) in communication with the controller, the controller activates the NFC receiver and transmitter 2 to read the information contained in the NFC Tag 11. The read information can be used by the injection device for the preparation of the medicament injection. The controller may further use the read information to check the type of medicament and e.g. the shelf life. If the expiration date is reached the injection device 1 may emit a signal (not shown) to the person who intends to use the device - therefore improving safety of the medicament use.

The NFC receiving and transmitting 2 device may be used to transmit data and further information to the smart phone 20 which comprises an appropriate reading unit (not shown). In order to transmit data and further information from the injection device 1 to the smart phone 20 the controller of the injection device 1 sets the NFC receiver and transmitter 2 into the so-called card emulation mode and provide the data and further information the NFC receiver and transmitter 2. The smart phone 20 and the injection device 1 are then moved to close proximity or directly adjacent which allows the reading unit of the smart phone to extract the data and further information from the NFC receiver and transmitter 2. The smart phone 20 is configured with software to analyze and process the received data and further information. For example transmitted error messages may be displayed on the display 21 of the smart phone 20. Moreover, the smart phone 20 is configured to forward at least part of the read data and further information, notably in processed form, to further devices, e. g. distant servers. This may be done through a local area network (LAN) connection, particularly through wireless LAN (WLAN) or a cellular or mobile network.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

- 1: autoinjector
- 1a: proximal outer housing
- 1b: distal outer housing
- 1c: inner housing
- 2: NFC receiver and transmitter
- 3: cartridge unit cavity

- 10: cartridge unit
- 10a: cartridge unit housing
- 11: NFC tag
- 12: prefilled syringe
- 13: syringe cannula
- 14: syringe piston
- 15: needle shield
- 16: needle shield removing unit

- 20: smart phone
- 21: display

## Claims

1. An injection device for injecting fluid medicaments comprising an electronic controller and functionally connected to the controller a near field communication unit,
the injection device further comprising a cartridge or cartridge unit with a cartridge adapted to contain the fluid medicament, wherein the cartridge/cartridge unit is preferably removably attachable to the injection device,
the cartridge or cartridge unit further comprising an near field communication readable item which stores information regarding the cartridge and/or the fluid medicament,
the near field communication readable item being readable by the near field communication unit to transfer the information,
**characterized in that**,
the near field communication unit is used in a first mode to read wirelessly the information, and
the near field communication unit is used in a second mode to wirelessly provide data to another device, which is a separate device, comprising a near field communication reading device.

2. An injection device according to claim 1, wherein near field communication is near field communication (NFC) according to the standard ISO/IEC 18092 and its normative references.

3. A system comprising an injection device according to any of the preceding claims and the other device.

4. A system according to the preceding claim, wherein the other device is a mobile computing device, preferably a smart phone or a PDA, more preferably a smart phone running Android OS as an operating system.

5. An injection device according to claims 1, 2 or a system according to claims 3 or 4, wherein the data provided by near field communication unit comprises error messages regarding the operation of the injection device and/or the injection device as such, or data about one or more states of the injection device, particularly error messages and/or data regarding the cartridge.

6. An injection device according to claims 1, 2 or 5, wherein the injection device is a modular injection device comprising a reusable module comprising the electronic controller and the near field communication unit and a disposable module comprising the cartridge or cartridge unit with the near field communication readable item, wherein the disposable module is removably attachable to the reusable unit, preferably through a snap, bayonet or threaded connection.

7. An injection device according to claim 6, wherein the injection device is an autoinjector further comprising a drive unit for providing injection force to the cartridge to force medicament out of the cartridge.

8. The injection device according to claims 1 or 7, the drive unit further comprising an energy storage, a movable driver assembly driven by energy out of the energy storage.

9. The injection device according to claim 8, wherein the energy storage is an electrical energy storage, preferably a battery, an accumulator or a super capacitor.

10. The injection device according to claim 9, wherein the driver assembly comprises an electric motor, optionally a gearing mechanism, a linearly moveable piston rod functionally connected to the electric motor, whereby activation of the electric motor induces a movement of the piston rod, wherein the cartridge comprises an internal volume for storing the medicament and a movable piston for changing the internal volume for provoking ejection of medicament, and wherein and if the disposable module is attached to the reusable module, movement of the piston rod in direction of the cartridge may move the piston and subsequently lead to ejection of medicament.

11. The injection device of any of the preceding claims, wherein the cartridge is a prefilled carpule or a prefilled syringe with a maximum filing volume for the medicament of 1 to 6 mL, preferably 1, 2.25, 4 or 6 mL.

12. The injection device of claim 11, wherein the cartridge is on partly prefilled.

13. The injection device of any of the preceding claims, wherein in the injection device further comprises an electronic display, preferable a LCD or OLED display.

14. The injection device of any of the preceding claims, wherein in the injection device further comprises at least one signaling device, preferably at least one LED for visually indicate states of or information regarding the injection device.

15. The injection device of claim 2, wherein the second mode of the near field communication (NFC) according to ISO/IEC 18092 and its normative references is an NFC card emulation mode or the NFC peer-to-peer mode.
